## Europäisches Patentamt

## European Patent Office

## Office européen des brevets

(11) Veröffentlichungsnummer: **0 157 271**
**B1**

(12) **EUROPÄISCHE PATENTSCHRIFT**

(45) Veröffentlichungstag der Patentschrifft:
07.02.90

(21) Anmeldenummer: 85103116.1

(22) Anmeldetag: 18.03.85

(51) Int. Cl. $^5$: **C 12 N 5/00**, **C 12 N 15/00**,
C 12 P 21/00, G 01 N 33/577,
A 61 K 39/395 //
(C12P21/00, C12R1:91)

(54) Hybridzell-Linie, Verfahren zu ihrer Herstellung und ihre Verwendung.

(30) Priorität: 16.03.84 CH 1333/84

(43) Veröffentlichungstag der Anmeldung:
09.10.85 Patentblatt 85/41

(45) Bekanntmachung des Hinweises auf die Patenterteilung:
07.02.90 Patentblatt 90/06

(84) Bennante Vertragsstaaten:
DE IT

(73) Patentinhaber: Byk Gulden Lomberg Chemische Fabrik GmbH
Byk-Gulden-Strasse 2
D-7750 Konstanz (DE)

(72) Erfinder: Rajewsky, Manfred F., Prof. Dr.
Elsassstrasse 88
D-4300 Essen (DE)
Erfinder: Kindler-Röhrborn, Andrea, Dr.
Fasanenstrasse 11
D-4300 Essen (DE)

(56) Entgegenhaltungen:

BIOLOGICAL ABSTRACTS, Band 73, Nr. 11, 1982, Seite 8037, Nr. 77044, Biological Abstracts, Inc., Philadelphia, PA., US; J.T. KEMSHEAD u.a.: "A monoclonal antibody detecting an antigen shared by neural and granulocytic cells" & PEDIATR. RES. 15(9): 1282-1286, 1981

NATURE, Band 291, Nr. 5814, 4. Juni 1981, Seiten 421-423, Macmillan Journals Ltd., Chesham, Bucks., GB; J. COHEN u.a.: "A neuronal cell-surface antigen is found in the CNS but not in peripheral neurones"

BIOLOGICAL ABSTRACTS, Band 75, Nr. 9, 1983, Seite 6845, Nr. 66145, Biological Abstracts, Inc., Philadelphia, PA., US; C.J. WIKSTRAN u.a.: "Human fetal brain antigen expression common to tumors of neuroectodermal tissue origin: Gliomas, neuroblastomas and melanomas" & J. NEUROIMMUNOL. 3(1): 43-62, 1982

(56) Entgegenhaltungen: (fortsetzung)

BIOLOGICAL ABSTRACTS, Band 73, Nr. 12, 1982, Seite 8829, Nr. 84537, Biological Abstracts, Inc., Philadelphia, PA., US; C.J. WIKSTRAND u.a.: "Expression of human fetal brain antigens by human tumors of neuroectodermal origin as defined by monoclonal antibodies" & CANCER RES. 42(1): 267-275, 1981

BIOLOGICAL ABSTRACTS, Band 76, Nr. 1, 1983, Seite 489, Nr. 4572, Biological Abstracts, Inc., Philadelphia, PA., US; J. RITZ u.a.: "Autologous bone-marrow transplantation in common acute lymphoblastic leukemia antigen positive acute lymphoblastic leukemia after in vitro treatment with J5 monoclonal antibody and complement" & LANCET 2(8289): 60-63, 1982

**Beschreibung**

Die Erfindung betrifft den monoklonalen Antikörper RB 21 - 7, welcher spezifisch an Gehirnzellen verschiedener Säugerspezies bindet und durch Züchtung der Zell-Line RB 21 - 7 (CNCM I-288) erhältlich ist, die Hybridzell-Linie mit der Bezeichnung RB 21 - 7 und der Hinterlegungsnummer I-288 (CNCM), die Verwendung der Hybridzell-Linie RB 21 - 7 und die Verwendung des monoklonalen Antikörpers RB 21 - 7.

Die Fusion von Zellen des Immunsystems mit Tumorzellen wurde von Köhler und Milstein (Nature 256 (1975) 495 - 97) beschrieben. Danach wird ein Lymphocyt, der Antikörper sezerniert, mit einer Tumorzelle fusioniert, wobei das gebildete Hybrid die Eigenschaften beider Ausgangszellen behält. Die Fähigkeit der unbegrenzten Teilungsfähigkeit wird von der Tumorzelle und die Fähigkeit, Antikörper eines bestimmten Typs zu sezernieren, wird vom Lymphocyten übernommen. Somit ist es möglich, die Antikörperantwort eines B-Lymphocyten gegen ein bestimmtes Immunogen auf Dauer zu erhalten und besonders interessante monoklonale Antikörper durch ständiges Kultivieren der sie sezernierenden Hybridzell-Linien herzustellen. Wegen ihrer Einheitlichkeit und Spezifität bedeuten monoklonale Antikörper eine Verbesserung gegenüber Antikörpergemischen (Antiseren) für diagnostische und therapeutische Zwecke. Die Herstellung von monoklonalen Antikörpern für den Nachweis von Tumorzellen wird z. B. im USP-4 172 124 beschrieben. In der EP-OS-90 343 wird die Herstellung eines Zellhybrids beschrieben, das monoklonale Antikörper sezerniert, die mit einem Bestandteil tierischer Zellen reagieren und die zur Therapie von Lungentumoren verwendet werden können.

Die Autoren J.T.Kemshead et al. (Biol.Abstr. Bd.73, Nr.11, 1982) diskutieren einen Antikörper M1/N1, der mit frischem Neuroblastomgewebe, Humanneuroblastomzell-Linien und Myeloidlinien reagiert. Die Untersuchungen der genannten Autoren zeigen jedoch, daß die Bindungsfähigkeit des genannten Antikörpers erheblichen quantitativen und qualitativen Schwankungen unterworfen ist.

Die Autoren C.J. Wikstrand et al. (Biol.Abstr.Bd.75, Nr.9, 1983) beschreiben verschiedene Antikörper, die an Neuroblastom- und fetale Gehirnzellen binden. Die genannten Antikörper haben keine Bindungsfähigkeit an das Gewebe des Gehirns von Erwachsenen.

Ein Antikörper A4, der einen Antikörper des IgM-Typs darstellt und an Neuronen von Ratten bindet, wird weiterhin von J.Cohen et al. (Nature 291 (1981) S.421 ff.) beschrieben. Dieser Antikörper bindet ausschließlich an Neuronen des zentralen Nervensystems bei Ratten und wurde dadurch hergestellt, daß Balb/c-Mäuse mit Ratten-Cerebellum-Zellen von 1 Woche alten Wistar-Furth-Ratten immunisiert wurden, gefolgt von der bekannten Hybridoma-Technik.

Durch Chemo- und Radiotherapie wird das Knochenmark von Patienten mit Tumorerkrankungen stark geschädigt. In Fällen mit metastasierenden Neuroplasmen, die man durch konventionelle Chemo- oder Radiotherapie behandeln will, müßte die Dosierung so hoch gewählt werden, daß sie für das Knochenmark und damit für den Patienten letal ist. Für solche Fälle wird die Transfusion von vor Therapiebeginn entnommenem autologen Knochenmark empfohlen. Neben der Kältekonservierung menschlichen Knochenmarks kommt es vor allem darauf an, möglichst viele für die Repopulation des Knochenmarks wichtige Zellen zu gewinnen, ohne gleichzeitig maligne Zellen miteinzuschleppen (Imbach et al., Schweiz. med.Wschr. 109 (1979) S. 283 - 287). Ein Mittel zur Eliminierung eventuell im Knochenmark vorhandener metastatischer Tumorzellen wäre wünschenswert. Die Transplantation von autologem Knochenmark nach in-vitro Behandlung mit einem monoklonalen Antikörper wird von Ritz et al. (Lancet, 10. Juli 1982, S. 60 - 63) beschrieben.

Überraschenderweise wurde nun gefunden, daß man durch Immunisierung von Mäusen mit pränatalen Gehirnzellen von Säugetieren eine Hybridzell-Linie erzeugen kann, die einen monoklonalen Antikörper sezerniert, der spezifisch an Gehirnzellen verschiedener Säugerspezies bindet, und zwar insbesondere an Neuronen des Cortex, Purkinje-Zellen des Cerebellum und Neuronen der äußeren Molekularschicht im Kleinhirn.

Außerdem bindet dieser Antikörper besonders stark an Zellen einer Vielzahl menschlicher Neuroblastome und ist für diese in Anwesenheit von Komplement cytotoxisch, wobei menschliche Knochenmarkszellen nicht angegriffen werden. Der Antikörper bindet im adulten menschlichen Großhirn an Neuronen des Cortex sowie an Purkinje-Zellen und Neuronen der äußeren Molekularschicht im adulten menschlichen Kleinhirn.

Eine weitere wesentliche Eigenschaft des von der erfindungsgemäßen Hybridzell-Linie sezernierten Antikörpers war die zusätzliche Bindungsfähigkeit an Rattengehirnzellen. Durch den weiten systematischen Abstand von Menschen und Ratten im Evolutionssystem war dieses breite Spektrum an Bindungsfähigkeit des erfindungsgemäßen monoklonalen Antikörpers völlig überraschend.

Gegenstand der Erfindung ist der monoklonale Antikörper RB 21 - 7, welcher spezifisch an Gehirnzellen verschiedener Säugerspezies bindet und durch Züchtung der Zell-Linie RB 21 - 7 (CNCM, I-288) erhältlich ist.

Gegenstand der Erfindung ist weiterhin die Hybridzell-Linie mit der Bezeichnung RB 21 - 7 und der Hinterlegungsnummer I-288 (CNCM) sowie deren Verwendung zur Herstellung des monoklonalen Antikörpers RB 21 - 7.

Ein weiterer Gegenstand der Erfindung ist der vorgenannte monoklonale Antikörper als Diagnostikum oder als Therapeutikum.

Weiterhin betrifft die Erfindung die Verwendung des monoklonalen Antikörpers RB 21 - 7 als Diagnostikum, ausgenommen in Diagnostizierverfahren, die am menschlichen oder tierischen Körper vorgenommen werden, sowie als Therapeutikum, insbesondere zur Säuberung von entnommenem Knochenmark.

Die erfindungsgemäße Hybridzell-Linie wird nach an sich bekannten Verfahren hergestellt. In diesem Verfahren werden in an sich bekannter Weise Mäuse mit pränatalen Gehirnzellen von Säugetieren immunisiert, Lymphocyten aus den immunisierten Mäusen gewonnen und mit Myelomzellen fusioniert und die Hybridzell-Linie isoliert.

Säugetiere, deren Gehirnzellen zur Immunisierung eingesetzt werden, sind u.a. Primaten, z. B. Mensch oder Affe, oder Nager, z. B. Maus, Hamster, Kaninchen, inbesondere Ratte.

B-Lymphocyten enthaltende Organe, z. B. Milz, Lymphknoten, oder peripheres Blut, der immunisierten Mäuse werden aseptisch entnommen und mit solchen Myelomzellen fusioniert, die in einem bestimmten Selektionsmedium nicht wachsen, z. B. X63-Ag8.653 in HAT-Medium.

Als Myelomzellen kommen solche verschiedenen Ursprungs in Frage, z. B. solche von Maus, Ratte oder Mensch. Bevorzugt sind Mäuse-Myelomzellen. Als Beispiele für verwendbare Myelomzell-Linien seien genannt P3-X63-Ag8 (Nature 256 (1975) S. 495), P3-NSI/1-Ag4-1 (Eur.J.Immunol.6 (1976) S. 292), Sp2/0 Ag14 (Nature 226 (1978) S. 269), insbesondere X63-Ag8.653 (J.Immun. 123 (1979) S. 1548). Die Zell-Linie X63-Ag8.653 ist beispielsweise beim Institut für Zellbiologie (Tumorforschung) der Universität Essen (GH) erhältlich. Sie ist ferner bei ATCC unter der No. CRL-1580 hinterlegt.

Die Fusion wird in an sich bekannter Weise durchgeführt, z. B. durch Mischen der Lymphocyten mit den Myelomzellen unter Zugabe eines bekannten Zellfusionsreagenz, beispielsweise Polyethylenglykol oder Sendaivirus.

Nach der Fusion werden die entstandenen Hybridzellen in einem Selektionszellkulturmedium, das zur Schaffung optimaler Bedingungen mit 20 % FCS komplementiert ist, kultiviert. Die Zellkulturüberstände der entstandenen Hybridklone werden auf ihren Gehalt an spezifischem Antikörper getestet. Die Zellkulturüberstände können mittels Radioimmunoassay (RIA), Enzyme-linked-Immunosorbent-Assay (ELISA) oder Immunfluoreszenztechnik an Zellmaterial, Extrakten oder gereinigtem Antigen auf spezifische Antikörper geprüft werden.

Die Hybridzellen, deren Zellkulturüberstände spezifische Antikörper gegen das zum Immunisieren verwendete Antigen enthalten, werden nochmals kloniert und erneut nach obigen Methoden auf ihre Fähigkeit überprüft, spezifische Antikörper zu sezernieren. Die Hybridzellen werden nach Abtrennung vom Überstand unter üblichen Bedingungen aufbewahrt, z. B. weiterkultiviert oder unter Bedingungen eingefroren, die ihre Rekultivierung nach Auftauen ermöglichen. Die Überstände werden auf Reaktivität mit anderen als dem spezifischen Antigen überprüft. Die Ergebnisse mit MAK RB 21 - 7 werden in Tabelle 1 wiedergegeben.

**Tabelle 1**

Bindungscharakteristika von MAK RB21 - 7 an verschiedene Zelltypen

| Humanzellen | Bindung |
| --- | --- |
| Neuroblastomzell-Linien Lan-1, Lan-5, CHP 134, SH, KA, Lan-4, IMR | +++ |
| Neuronen (Cortex) | +++ |
| Purkinje-Zellen (Cerebellum) | +++ |
| Neuronen der äußeren Molekularschicht | +++ |
| Neuroblastomzell-Linien CHP100 und MC | + |
| Coloncarcinomzell-Linie HT29 | – |
| Blasencarcinomzell-Linie EJ | – |
| Osteogene Sarcomzell-Linie H1080 | – |
| Hämatopoetische Stammzellen | – |

| Rattenzellen (BDIX-Ratte) | |
| --- | --- |
| Leberzellen | – |
| Bindegewebszellen | – |
| Gehirnzellen* | |
| a) Neuronen (Neurofilament positiv) | + |
| b) Vimentin-positive Vorläuferzellen von Neuronen | + |
| c) Vimentin-positive Vorläuferzellen von Astrozyten | + |
| Subklone der Zell-Linien BT1C-BT15C** | – |
| Subklone der Zell-Linien GV1C, TV1C, NV1C*** | – |

+++     sehr starke Bindung
+     schwache Bindung
-     keine Bindung

*)     praenatale Gehirnzellen; die MAK RB21 - 7 bindende Zell-Subpopulation ist ab 13. Tag der Pränatalentwicklung nachgewiesen (13. Tag: 16 %, 14. Tag: 32 %, 15. Tag: 59 %, 16. Tag: 86 %), ab dem 17. Pränataltag nimmt dann nur, noch die Zahl der antigenen Determinanten zu, nicht aber der Anteil der von MAK RB21 - 7 erkannten Gehirnzellen.

**)     maligne oder praemaligne neuroektodermale Zell-Linien (O.D. Laerum u.M.F.Rajewsky J.Natl.Cancer Inst. 55 (1975) S1177 - 87) entstanden durch Transformation in vitro nach Einwirkung von Ethylnitrosoharnstoff auf fetale Rattengehirnzellen in vivo.

***)     maligne Zell-Linien, abgeleitet von durch Ethylnitrosoharnstoff induzierten Tumoren des Nervensystems der BDIX-Ratte.

Der von der kultivierten Hybridzell-Linie sezernierte monoklonale Antikörper MAK RB 21 - 7 kann als solcher zur Diagnose von normalen Zellen oder malignen entarteten Neuroblasten und zur Therapie von neuralen Tumoren verwendet werden.

Weiterhin kann der monoklonale Antikörper MAK RB 21 - 7 zur Neuron-Lokalisierung, vorzugsweise zur Neuroblastom-Lokalisierung, mit einem Indikator, z. B. $^{111}$In, $^{99m}$TC, $^{125}$I, markiert werden, um als Diagnostikum eingesetzt zu werden (vgl. Eckelmann, W. et al. Cancer Res. 40 (1980) S. 3036 - 3042, Radiolabelling of Antibodies).

Für therapeutische Zwecke kann der monoklonale Antikörper MAK RB 21 - 7 als Transportmolekül für cytotoxische Substanzen dienen, welche mittels chemischer Methoden oder über Liposomen indirekt an den Antikörper gekoppelt sind.

In der Therapie besitzt der erhaltene Antikörper MAK RB 21 - 7, besondere Bedeutung für die autologe Knochenmark-Reimplantation aufgrund seiner Fähigkeit, an menschliche Neuroblastomzellen zu binden und diese Zellen in Gegenwart von Komplement zu lysieren. Dabei wird der Antikörper dazu benutzt, Tumorzellen im entnommenen Knochenmark zu zerstören, d.h. das Knochenmark, bevor es wieder infundiert wird, von malignen Zellen zu säubern. Dazu wird die Knochenmarksprobe mit Antikörper enthaltender Lösung inkubiert, z. B. bei 0 - 40°C, Komplement hinzugefügt und einige Zeit stehen gelassen, z. B. 20 bis 40 Minuten; die Suspension wird zentrifugiert, anschließend werden die Knochenmarkszellen mit Puffer-Lösung, z. B. PBS, gewaschen. Gegebenenfalls wird anstelle der Antikörper enthaltenden Lösung ein Antikörper-Cocktail, d.h. eine den erfindungsgemäßen und weitere Antikörper enthaltende Lösung, eingesetzt.

Die folgenden Beispiele dienen zur näheren Erläuterung der Erfindung. Eine Erklärung der verwendeten Abkürzung folgt auf Seite 15.

## Beispiel 1

Zur Immunisierung wurden 8 Wochen alten weiblichen Balb/c-Mäusen jeweils 2 x $10^7$ mechanisch dissozierte, fetale (21. Tag der Praenatalentwicklung) Gehirnzellen von Ratten des Inzuchtstammes BDIX (in 1 ml PBS) intraperitoneal an den Tagen 0 und 21 injiziert. Am Tag 42 wurden die gleichen Zellen in gleicher Anzahl intravenös injiziert. Am Tag 46 wurden die Milzzellen der immunisierten Tiere aseptisch entnommen, mittels eines Siebes zerkleinert und in Zellkultur-Medium RPMI 1640 ohne Serum aufgenommen. Die Zahl der Lymphocyten wurde mittels einer Zählkammer bestimmt.

Parallel hierzu wurden in Gewebekultur gehaltene Mausmyelomzellen vom Stamm X63-Ag8.653 in serumfreiem RPMI aufgenommen und die Zahl der Tumorzellen mittels einer Zählkammer ermittelt.

Zur Fusion wurden die Maus-Myelomzellen (2,2 x $10^8$ Zellen) mit den Lymphocyten (2,2 x $10^8$ Zellen) gemischt, zentrifugiert (10 Minuten, 95xg) und zum Pellet 1,5 ml 50 %-ige PEG-Lösung (PEG: Molgewicht 4000) in 45 Sekunden zugesetzt. Unter leichtem Schütteln wurden die Zellaggregate dissoziert und sofort 10 ml RPMI innerhalb 7 Minuten tropfenweise zugegeben, danach wurden innerhalb 7 Minuten weitere 40 ml RPMI zugesetzt.

Nach 10 Minuten Zentrifugation bei 55xg wurde das Sediment so in RPMI (+20 % FCS) verdünnt, daß 2 x $10^5$ Zellen pro Vertiefung einer Zellkultur-Platte mit 24 Vertiefungen á 2 cm$^2$ ausgesät werden konnten. Nachdem die Hybridklone soweit herangewachsen waren, daß sie den Boden der Löcher total bedeckten, wurde von jeder Kultur die einen wachsenden Zell-Klon enthielt, 50 µl Überstand entnommen und der Hybridom-Überstand auf seinen spezifischen Antikörpergehalt überprüft.

Die Prüfung der Hybridzell-Klone auf Produktion monoklonaler Antikörper, die gegen Strukturkomponenten der Zelloberflächen der zur Immunisierung verwendeten neuralen Rattengehirnzellen gerichtet sind, erfolgte durch einen indirekten solid phase radioimmunoassay (RIA) auf Plasmamembranen von fetalen Rattengehirnzellen (21. Tag der Praenatalentwicklung) nach der Methode von Howard et al. (J.Immunol.Meth.38 (1980)s.75 - 82).

Hybridzell-Klone, deren Kulturüberstände im RIA eine spezifische Bindung an Plasmamembranen zeigten, wurden weiter kultiviert. Durch indirekte Immunfluoreszenz (Methode s.u.) an fetalen Gehirnzellen (21. Tag der Praenatalentwicklung) in Primärkultur werden diejenigen Hybridzell-Klone selektioniert, die gegen die Außenseite der Zellmembranen gerichtete monoklonale Antikörper sezernieren. Diese Hybridzell-Klone wurden noch 3 mal rekloniert. Eine Zell-Linie, deren Antikörper an menschliche Neuroblastomzellen bindet, stellt die Hybridzell-Linie RB21 - 7 dar. Eine Probe dieser Hybridzell-Linie wurde hinterlegt beim Institut Pasteur (CNCM), Hinterlegungsnummer I-288.

Indirekte Immunfluoreszenzmessungen an kultivierten praenatalen Gehirnzellen wurden wie folgt durchgeführt: Die praenatalen Rattengehirne wurden mit Hilfe einer kombinierten enzymatisch-mechanischen Technik dissoziiert (R.Müller und M.F.Rajewsky: Cancer Res.43 (1983)S.2897 - 2904). 5 x $10^5$ Zellen wurden auf mit Poly-L-Lysin beschichteten Deckgläsern (13 mm Durchmesser) in Eagle-Medium FM (Modifikation nach Frank) mit einem Zusatz von 10 % FCS ausgesät. Man ließ die Zellen 24 Stunden wachsen, wusch mit FM-Medium, dem 0,1 % BSA zugefügt war, und inkubierte 10 Minuten mit 40 µl des Hybridzell-Überstandes. Nach zweimaligem Waschen mit FM, dem 0,1 % BSA zugefügt war, wurden die Zellen 5 Minuten in PBS mit 4 % Paraformaldehyd fixiert und zweimal mit CMF-PBS, dem 0,1 % BSA zugefügt war, gewaschen. Die fixierten Zellen wurden 20 Minuten mit 40 µl Schaf-F(AB)$_2$-anti-Maus-Ig, das mit Fluorescein markiert ist, inkubiert. Nach zweimaligem Waschen mit CMF - PBS, dem 0,1 % BSA zugefügt war, wurden die Präparate mit 90 %-iger Glycerinlösung in PBS unter Zusatz von 0,1 % p-Phenylendiamin auf Objektträgern eingedeckt und im Fluoreszenzmikroskop untersucht. Alle Verfahrensschritte erfolgten bei Raumtemperatur (ca. 20°C).

RB21 - 7 wird in folgendem Milieu (M) kultiviert:
RPMI 1640 unter Zusatz von

| 100 I.U. | Penicillin/ml |
|---|---|
| 100 I.U. | Streptomycin/ml |
| 2,5 µg | Amphotericin B/ml |
| 0,055 mM | 2-Mercaptoethanol |

Die Züchtung erfolgt in 20 %-igem FCS bei 37°C. Zur Daueraufbewahrung werden $3 \times 10^6$ Zellen/ml in M unter Zusatz von 20 % fetalem Kälberserum und 20 % Dimethylsulfoxid eingefroren.

Der durch RB21 - 7 sezernierte monoklonale Antikörper MAK RB 21 - 7 (Isotyp: IgM) hat die folgenden Eigenschaften: Er bindet im praenatalen Rattengehirn an eine Zellpopulation, die sich in ihrem Anteil an der Gehirnzellgesamtpopulation bis zum 16. Tag der Praenatalentwicklung ständig vergrößert. Diese Ergebnisse wurden durch indirekte Immunfluoreszenz im fluoreszenzaktivierten elektronischen Zellsorter (FACS) ermittelt und, was die mittlere Zahl der Antigendeterminanten pro MAK RB21 - 7 bindende Zellen angeht, durch RIA auf Gehirnzell-Membranen aufeinanderfolgender Tage der Praenatalentwicklung bestätigt. MAK RB21 - 7 bindet nicht an Leber- und Bindegewebs-Zellmembranen der BDIX-Ratte und nicht an die malignen und praemalignen neuroektodermalen BDIX-Rattenzell-Linien BT1C -BT15C, GV1C, NV1C, TV1C (siehe Tabelle). MAK RB21 - 7 bindet an menschliche Neuroblastomzellen und ist in Anwesenheit von Komplement für diese cytotoxisch, jedoch nicht für menschliche Knochenmarkszellen. MAK RB21 - 7 bindet im adulten Cerebrum des Menschen an Neuronen des Cortex sowie an Purkinjezellen und Neuronen der äußeren Molekularschicht im adulten menschlichen Cerebellum.

**Beispiel 2**

*Züchtung von MAK RB21 - 7 in vitro:* Ca. $2 \times 10^6$ Hybridzellen werden in einer Kulturflasche mit 150 m$^2$ Grundfläche in 50 ml M ausgesät. Wenn sich nach 3 - 4 Tagen ein dichter Zellrasen gebildet hat, wird das gesamte abgenommen und bei 320 x g abzentrifugiert. Es enthält ca. 10 µg MAK RB21 - 7/ml. Die Hälfte der Hybridzellen wird durch Abspülen mit neuem M gewonnen und entweder auf eine zweite Flasche ausgesät oder verworfen. Die Ursprungskultur wird wieder mit neuem M aufgefüllt. Aus einer 150 cm$^2$ Kulturflasche werden 2 x wöchentlich 50 ml MAK RB21 - 7 enthaltender Zellkulturüberstand gewonnen.

*Züchtung von MAK RB21 - 7 in vivo:* 0,5 ml Pristan werden 6 - 8 Wochen alten Balb/c-Mäusen intraperitoneal injiziert. Nach 7 - 9 Tagen werden $5 \times 10^5$ Hybridzellen in die Peritonealhöhle gespritzt. Nach weiteren 8 - 10 Tagen werden die Tiere durch cervicale Dislokation getötet. Die Ascitesflüssigkeit (6 - 10 ml/Tier) wird durch Kanülierung der Bauchhöhle gewonnen. Nach Zentrifugation bei 320 x g enthält der Überstand ca. 1 - 1,5 mg MAK RB21 - 7/ml.

**Beispiel 3**

MAK RB21 - 7 kann als Diagnostikum analog Ionak, Z.L. (Hybridoma 1/4 (1982)S.349 - 368) eingesetzt werden. Gewebsschnitte (Gefrierschnitte) oder cytologisches Material (Cytozentrifugenpräparat) werden zuerst mit MAK RB21 - 7, dann mit normalem Kontrollserum inkubiert (je 20 Minuten bei Raumtemperatur). Die Präparate werden 3 mal mit PBS gewaschen, anschließend mit biotinyliertem Anti-Maus-Ig weitere 10 Minuten inkubiert, und danach wieder 3 mal mit PBS gewaschen. Anschließend wird 10 Minuten mit Avidin-Biotin-Peroxidase-Komplex inkubiert und wiederum 3 mal mit PBS gewaschen, dann unter Zugabe von H$_2$O$_2$ mit oxidierbarem Peroxidase-Substrat inkubiert und nochmals 3 mal mit PBS gewaschen. Die nachzuweisenden RB21 - 7 positiven Zellen sind an ihrer Färbung erkennbar.

Entsprechend kann die MAK RB21 - 7-Bindung durch einen fluoreszenzgekoppelten Anti-Maus-Ig-Antikörper analog Kemshead, I.T. et al. (Lancet 1 (1983)S.8314 - 15) nachgewiesen werden.

**Beispiel 4**

Die Markierung von MAK RB21 - 7 mit $^{125}$I wird nach der Chloramin-T-Methode (Hunter and Greenwood, Nature 194 (1962)S. 495) vorgenommen.

Die Jodmarkierung kann auch nach Bolton und Hunter (Biochem.J. 133 (1973)S.529), nach der Lactoperoxidase-Methode (Machalonis, Biochem.J. 113 (1969)S.299) oder nach der Iodmonochlorid-Methode (Mac Fahrland, Nature 182 (1958)S.53) erfolgen.

**Beispiel 5**

*Komplementabhängige Lysis von Tumorzellen in vitro*

Die zu testenden Zellen werden in RPMI 1640 für die Dauer von 24 Stunden kultiviert und anschließend mit $Na_2{}^{51}CrO_4$ ($50\mu Ci/10^6$ Zellen) 15 - 18 Stunden unter den gleichen Kulturbedingungen markiert. Die Zellen werden mit EDTA-haltigem CMF - PBS und Glucose abgelöst, zweimal mit PBS gewaschen und wieder in RPMI + 5 % FCS aufgenommen. 2 x $10^4$ Zellen in 50 µl RPMI werden in die U-förmigen Vertiefungen einer 96-Loch-Mikrotiterplatte verteilt und mit 75 µl Hybridzellüberstand (enthaltend MAK RB21 - 7) bzw. RPMI 5 % FCS als Kontrolle 2 Stunden lang bei Raumtemperatur inkubiert. Die Zellen werden einmal mit 100 µl RPMI gewaschen und dann mit 50 µl einer 1 : 20 Verdünnung von Kaninchenkomplement bzw. RPMI als Kontrolle 1 Stunde bei 37°C in einem mit 5 % $CO_2$ begasten Brutschrank inkubiert. Zu jeder Probe werden 100 µl RPMI hinzugegeben. Die Mikrotiterplatte wird bei 200xg 10 Minuten zentrifugiert. Die Hälfte des Überstandes wird zur Bestimmung des freigesetzten $Na_2{}^{51}CrO_4$ in einem $\gamma$-Zähler verwendet. Die maximal mögliche Freisetzung von $Na_2{}^{51}CrO_4$ wird durch Inkubation der markierten Zellen in PBS + 1 % Triton über Nacht bei Raumtemperatur bestimmt. Der Prozentsatz der spezifischen Komplement-abhängigen Zell-Lyse wird berechnet nach:

$$100 \times \frac{\text{cpm, freigesetzt durch den Hybridzellüberstand} - \text{cpm, freigesetzt durch Mediumkontrolle}}{\text{maximal freigesetzte cpm} - \text{cpm, freigesetzt durch Mediumkontrolle}}$$

**Beispiel 6**

In vitro-Behandlung von Knochenmark zur Eliminierung von metastatischen Tumorzellen

Die Knochenmarkszellen werden bei einer Konzentration von 2 x $10^7$ Zellen/ml dreimal hintereinander einer Behandlung mit MAK RB21 - 7 (1 : 100 Verdünnung aus Ascites) und mit Kaninchen-Komplement (1 : 10 Verdünnung) unterzogen. Sie werden jeweils für 15 Minuten bei 4°C mit MAK RB21 - 7 und bei 37°C 30 Minuten mit Komplement inkubiert und nach jeder Behandlung zweimal mit RPMI 1640 gewaschen. Um die Effektivität des Verfahrens zu überprüfen, werden $^{51}$Cr markierte antigenpositive Tumorzellen einer Kontrollprobe von Knochenmark zugesetzt, die gleich behandelt wird.

*Liste der verwendeten Abkürzungen*

| | |
|---|---|
| BSA | bovine serum albumin |
| CMF - | calcium, magnesium free - |
| cpm | counts per minute |
| ELISA | Enzym-linked-Immunoadsorbens-Assay |
| EDTA | ethylenediaminetetraacetic acid |
| FCS | fetal calf serum |
| FM | Franksche Modifikation von Eagle's Medium (Frank et al. Experimentell Cell Res. 70 (1972)S.390 - 396) |
| HAT | Hypoxanthin, Aminopterin, Thymidin |
| Ig | Immunoglobulin |
| M | Milieu (RPMI 1640 + Zusätze) |
| MAK | monoklonaler Antikörper |
| PBS | phosphate buffered saline (NaCl 8.000 g $Na_2HPO_4$ 1.150 g KCl 0.200 g $MgCl_2$ 0.047 g $KH_2PO_4$ 0.200 g $CaCl_2$ 0.200 g Aqua bidest.ad 1 Liter) |
| PEG | polyethylene glycol |
| RIA | radioimmunoassay |
| RPMI 1640 | Rosewell Park Memorial Institute (Medium 1640) (Culture of Normal Human Leucocytes, Moore et al. J.A.M.A. 199 (1967)S.519 - 524) |

**Patentanspüche**

1. Monoklonaler Antikörper RB 21 - 7, welcher spezifisch an Gehirnzellen verschiedener Säugerspezies bindet, erhältlich durch Züchtung der Zell-Linie RB 21 - 7 (CNCM, I-288).

2. Hybridzell-Linie mit der Bezeichnung RB 21 - 7 und der Hinterlegungsnummer I-288 (CNCM).

3. Verwendung der Hybridzell-Linie RB 21 - 7 nach Anspruch 2 zur Herstellung des monoklonalen Antikörpers RB 21 - 7.

4. Monoklonaler Antikörper nach Anspruch 1 zur Verwendung als Diagnostikum.

5. Monoklonaler Antikörper nach Anspruch 1 zur Verwendung als Therapeutikum.

6. Verwendung des monoklonalen Antikörpers nach Anspruch 1 als Diagnostikum, ausgenommen in Diagnostizierverfahren, die am menschlichen oder tierischen Körper vorgenommen werden.

7. Verwendung des monoklonalen Antikörpers nach Anspruch 1 zur Säuberung von entnommenem Knochenmark.

## Claims

1. Monoclonal antibody RB 21 - 7, which binds specifically to brain cells of various mammalian species, obtainable by cultivation of the cell line RB 21 - 7 (CNCM I-288).

2. Hybrid cell line having the designation RB 21 - 7 and the depository number I-288 (CNCM).

3. Use of the hybrid cell line RB 21 - 7 according to claim 2 for the preparation of the monoclonal antibody RB 21 - 7.

4. Monoclonal antibody according to claim 1 for use as a diagnostic agent.

5. Monoclonal antibody according to claim 1 for use as a therapeutic agent.

6. Use of the monoclonal antibody according to claim 1 as a diagnostic agent, except in diagnostic procedures which are performed on the human or animal body.

7. Use of the monoclonal antibody according to claim 1 for the purification of collected bone marrow.

## Revendications

1. Anticorps monoclonal RB 21 - 7, qui se fixe spécifiquement sur les cellules cérébrales de différentes espèces mammifères, pouvant être obtenu par culture de la lignée cellulaire RB 21 - 7 (CNCM I-288).

2. Lignée de cellules hybrides ayant la désignation RB 21 - 7 et le numéro de dépôt I-288 (CNCM).

3. Utilisation de la lignée de cellules hybrides RB 21 - 7 selon la revendication 2 pour la préparation de l'anticorps monoclonal RB 21 - 7.

4. Anticorps monoclonal selon la revendication 1 pour l'utilisation comme agent de diagnostic.

5. Anticorps monoclonal selon la revendication 1 pour l'utilisation comme agent thérapeutique.

6. Utilisation de l'anticorps monoclonal selon la revendication 1 comme agent de diagnostic, excepté dans les procédés de diagnostic qui sont mis en oeuvre sur le corps humain ou le corps animal.

7. Utilisation de l'anticorps monoclonal selon la revendication 1 pour la purification de la moelle osseuse prélevée.